# EUROPÄISCHE PATENTANMELDUNG

(11) **EP 3 091 456 A1**
(43) Veröffentlichungstag der Anmeldung: **09.11.2016**
(21) Anmeldenummer: 15001359.7
(22) Anmeldetag: 07.05.2015
(51) Int. Cl.: G06F 19/00

(54) **SYSTEM ZUR ERFASSUNG MEDIZINISCHER DATEN**

(71) Anmelder: Deutsche Telekom AG, 53113 Bonn (DE)
(72) Erfinder: HEINZ, Sebastian, 53227 Bonn (DE); WINDHEUSER, Jörg, 40627 Düsseldorf (DE); SCHMIDT-GABRIEL, 82234 Wessling (DE); BEER, Andreas, 85630 Grasbrunn (DE)
(74) Vertreter: Cohausz Hannig Borkowski Wißgott

(57) **Zusammenfassung**

System zur Erfassung medizinischer Daten von einem Nutzer umfassend eine, bevorzugt stiftförmige Vorrichtung mit wenigstens zwei in einer Erstreckungsrichtung, bevorzugt Längserstreckungsrichtung hintereinander liegenden Komponenten, wobei eine Komponente zumindest ein Kommunikationsmodul umfasst und eine Komponente, insbesondere eine endseitig angeordnete Komponente ein bevorzugt auswechselbares Datenerfassungsmodul umfasst, mittels dem Daten, insbesondere gesundheitsspezifische Daten vom Körper des Nutzers erfassbar sind und umfassend wenigstens eine Datenbank auf einer in einem Kommunikationsnetzwerk angeordneten Datenverarbeitungsanlage, insbesondere an der das Kommunikationsmodul anmeldbar oder fix angemeldet ist, wobei die Vorrichtung eingerichtet ist, von dem Datenerfassungsmodul erfasste Daten mittels des Kommunikationsmoduls an die wenigstens eine Datenbank zu übertragen.

## Beschreibung

Die Erfindung betrifft ein System zur Erfassung medizinischer Daten von einem Nutzer. Systeme dieser Art sind im Stand der Technik bekannt, beispielsweise als Geräte, die von einem Nutzer bzw. Patienten jederzeit mitgeführt werden können, um gesundheitsspezifische Daten vom Körper des Nutzers zu erfassen.

Beispielhaft seien Blutdruckmessgeräte genannt oder auch mobile Geräte zur Analyse von Bluttropfen hinsichtlich einiger speziell interessierender Werte, wie z.B. Blutzucker oder Blutfette.

Solche Geräte ermöglichen es, einem Nutzer seinen Körper bzw. spezielle Körperfunktionen z.B. kontinuierlich zu überwachen und bei festgestellten Abweichungen von gewünschten Werten korrigierend einzugreifen.

Dies setzt voraus, dass der Nutzer entsprechend informiert ist über die gewünschten Richtwerte, so dass er auch in die Lage versetzt ist, Abweichungen entsprechend qualifiziert zu erkennen und die nötigen Maßnahmen zu ergreifen. Dabei ist davon auszugehen, dass solche Kenntnisse im Wesentlichen nur bei chronisch kranken Personen bekannt sind, wie z.B. Diabetes-Patienten.

Im Stand der Technik sind weiterhin Geräte oder Systeme bekannt, die z.B. selbsttätig Blutanalysen vornehmen und unmittelbar dem Nutzer ein Ergebnis der Analyse anzeigen, was als bedenklich einzustufen ist, da ein Nutzer hierdurch negative Informationen erfahren kann, die im einfachen Fall zu Besorgnis, gegebenenfalls zu panischem Fehlverhalten oder auch falscher Eigentherapie führen können, so dass erst hierdurch gesundheitlich bedenkliche Folgen resultieren.

Es ist daher eine Aufgabe der Erfindung, ein System und auch eine Vorrichtung zur Erfassung medizinischer Daten bereitzustellen, durch deren Nutzung ein Nutzer, insbesondere Patient, in die Lage versetzt ist, seinen Körper oder Körperfunktionen zu jeder von ihm gewünschten Zeit einer Überwachung zu unterziehen, wobei jedoch sicher gestellt bleibt, dass der Nutzer nicht zu eigenen Fehldiagnosen verleitet wird, aus denen gegebenenfalls ein nachfolgendes Fehlverhalten resultiert, sondern wodurch sicher gestellt wird, dass ein Nutzer nur im Fall von tatsächlich erkannter medizinischer Relevanz kontaktiert wird durch entsprechend qualifizierte Personen, also beispielsweise Ärzte.

Insbesondere soll ein System oder eine Vorrichtung bereitgestellt werden, welche keinerlei diagnostische Funktionalität aufweist, um technisch bedingte Fehldiagnosen auszuschließen.

Die Aufgabe wird gelöst durch ein System zur Erfassung medizinischer Daten von einem Nutzer umfassend eine, bevorzugt stiftförmige Vorrichtung mit wenigstens zwei in einer Erstreckungsrichtung, bevorzugt Längserstreckungsrichtung hintereinander liegenden Komponenten, wobei eine Komponente zumindest ein Kommunikationsmodul umfasst und eine Komponente, insbesondere eine endseitig angeordnete Komponente ein bevorzugt auswechselbares Datenerfassungsmodul umfasst, mittels dem Daten, insbesondere gesundheitsspezifische Daten vom Körper des Nutzers erfassbar sind und wobei das System weiterhin eine Datenbank umfasst, auf einer in einem Kommunikationsnetzwerk (z.B. Internet) angeordneten Datenverarbeitungsanlage, wobei die Vorrichtung eingerichtet ist, von dem Datenerfassungsmodul erfasste Daten mittels des Kommunikationsmoduls an die wenigstens eine Datenbank zu übertragen, z.B. durch Mobilfunk.

Hier ist es ein wesentlicher Kerngedanke der Erfindung, dass zwar ein Nutzer mit einem solchen System bzw. der darin vorhandenen Vorrichtung zur Erfassung medizinischer Daten in die Lage versetzt ist, seinen Körper bzw. Körperfunktionen jederzeit medizinisch dadurch zu überwachen, dass er entsprechende Daten von seinem Körper erfasst, diese Daten jedoch nicht einer Auswertung auf der Vorrichtung, die zur Erfassung dient, unterliegen, sondern die Daten ungeändert, insbesondere ohne jegliche Auswertung an eine Datenbank übertragen werden, wo diese Daten zunächst gespeichert werden.

Beispielsweise kann es hier vorgesehen sein, dass das Kommunikationsmodul der eingesetzten Vorrichtung an einer solchen Datenbank bzw. der diese verwaltenden Datenverarbeitungsanlage anmeldbar ist oder gegebenenfalls sogar herstellerseitig fix angemeldet ist.

Eine solche Anmeldung kann z.B. dadurch gegeben sein, dass in dem Kommunikationsmodul eine Netzwerkadresse hinterlegt ist, unter welcher die Datenbank bzw. die verwaltende Datenverarbeitungsanlage erreichbar ist, so dass dem Kommunikationsmodul hierdurch bekannt ist, an welche Adresse im Netzwerk die Daten zu versenden sind, z.B. durch Mobilfunk, insbesondere via GSM.

Auf der Datenverarbeitungsanlage bzw. der verwalteten Datenbank kann ein Nutzer z.B. ein Nutzerkonto unterhalten, so dass innerhalb dieses Nutzerkontos die von dem Kommunikationsmodul eingehenden Daten speicherbar sind. Es besteht auch die Möglichkeit ,die Daten anonym ohne Nutzerkonto an die Datenbank zur Verfügung zu stellen.

Eine solche Datenbank kann z.B. eine zentrale Datenbank sein, an welcher mehrere Vorrichtungen zur Erfassung medizinischer Daten der vorgenannten Art von mehreren verschiedenen Nutzern gleichzeitig angemeldet sein können. Eine solche Datenbank oder auch mehrere solcher Datenbanken können z.B. bei einem sogenannten Trust Center gehostet sein.

Konstruktiv kann das System bzw. die Vorrichtung vorteilhaft dadurch weitergebildet sein, dass eine dem Datenerfassungsmodul, welches bevorzugt auswechselbar ist, endseitig gegenüber liegende Komponente eine Energieversorgung bildet. Bevorzugt kann auch diese Energieversorgung eine von mehreren hintereinander liegenden Komponenten bilden, die jederzeit von dem Nutzer ausgetauscht werden kann, so dass der Nutzer auch in die Lage versetzt ist, mehrere solche Energieversorgungen mit sich zu führen, um eine allzeitige Bereitschaft der Vorrichtung sicherzustellen.

Verschiedene auswechselbare Datenerfassungsmodule können an der Vorrichtung bzw. in Verbindung mit dem System vorgesehen sein, um auf unterschiedliche Art und Weise Daten vom Körper zu erfassen.

Beispielsweise kann ein Datenerfassungsmodul ausgebildet sein zur Erfassung von Bildern, also beispielsweise eine Kamera umfassen, mittels der Körperregionen, insbesondere Hautareale bildlich erfasst werden können.

Es kann auch vorgesehen sein, Datenerfassungsmodule zur Erfassung von Urinwerten, zur Erfassung von Blutwerten, zur Erfassung von Blutdruckwerten oder zur Erfassung von Ultraschallwerten bereitzustellen.

Ebenso besteht die Möglichkeit, Datenerfassungsmodule für andere nicht hier genannte Erfassungsarten bzw. Untersuchungsmethoden bereitzustellen.

In einer vorteilhaften Weiterbildung des Systems ist dieses eingerichtet, eine Begutachtung der erfassten und an die wenigstens eine Datenbank übertragenen Daten durch eine qualifizierte Person, insbesondere einen Arzt, abzuwarten.

Hier ist darauf hinzuweisen, dass eine Auswertung der Daten auch in diesem Fall nicht automatisiert durch die Datenverarbeitungsanlage erfolgt, sondern die Daten mit menschlicher Qualifikation begutachtet werden, um aus den Daten Rückschlüsse zu ziehen, beispielsweise ein im Sinne des Patienten positives Ergebnis bereit zu erstellen, dass also der Patient nicht krank ist und keinerlei Handlungsanweisungen zu geben sind oder aber auch ein negatives Ergebnis der Begutachtung zu erstellen, wobei ein negatives Ergebnis im Sinne der Erfindung dann vorliegt, wenn eine qualifizierte Person, insbesondere ein Arzt die Notwendigkeit erkennt, dem Nutzer Handlungsanweisungen zukommen zu lassen. Dies muss nicht zwingend bedeuten, dass der Patient aktuell erkrankt ist.

Die Erfindung kann es vorsehen, dass die Datenbank bzw. Datenverarbeitungsanlage des Systems sich solange passiv verhält, bis ein etwaiges Ergebnis der Begutachtung von der qualifizierten Person vorliegt, ggfs. aber nicht länger als ein vorbestimmtes Zeitintervall.

Die Erfindung kann sodann vorsehen, dass nur bei einem im Sinne des Nutzers positiven Gutachten, das also keinerlei Handlungsanweisungen zu geben sind und keine gesundheitliche Beeinträchtigung festgestellt ist, an die Vorrichtung eine das positive Ergebnis signalisierende Nachricht gesendet wird.

Hierdurch wird sichergestellt, dass ein Nutzer nach Erhalt dieser Nachricht, die z.B. an der Vorrichtung signalisiert werden kann, z.B. durch eine optische oder akustische oder sonstige Anzeige hinsichtlich seines Gesundheitszustandes beruhigt ist.

Bei einem eventuellen negativen Ergebnis einer Begutachtung durch die qualifizierte Person kann es die Erfindung besonders vorteilhaft vorsehen, dass ein solches Ergebnis dem Nutzer nur mittels einer Kommunikationsverbindung zwischen dem Nutzer und der qualifizierten Person, insbesondere einem Arzt übermittelt wird, so dass der Nutzer konkret von dem Arzt Informationen zum Ergebnis der Begutachtung erhalten kann, beispielsweise Handlungsanweisungen oder auch beruhigende Worte, wenn eine Erkrankung festgestellt ist.

In besonders bevorzugter Ausführung kann es hier vorgesehen sein, dass eine sprachbasierte Kommunikationsverbindung zwischen dem Nutzer und dem Arzt hergestellt wird, oder in einfacher aber auch kombinierbarer Ausführung eine textbasierte Kommunikationsverbindung, wobei die Erfindung vorsehen kann, dass bei einer textlichen Übermittlung von Informationen diese Informationen konkret von der begutachtenden qualifizierten Person einzugeben sind, somit keine in der Datenbank hinterlegten Standardanweisungen oder sonstiges übermittelt wird, so dass ein Patient bzw. Nutzer jederzeit darauf vertrauen darf, eine individuelle Beratung über das System von einem Menschen zu erhalten.

Für die Zwecke einer Sprachkommunikation zwischen der qualifizierten Person, insbesondere dem Arzt sowie dem Nutzer kann das System vorsehen, dass eine Telefonnummer des Nutzers im System, insbesondere der Datenbank hinterlegt ist, so dass die qualifizierte Person (Arzt) über diese Telefonnummer die Kommunikationsverbindung zu einem Kommunikationsgerät des Nutzers erstellen kann.

In einer möglichen Ausführung kann es auch vorgesehen sein, dass die Vorrichtung zur Erfassung von Daten ein Kommunikationsmodul aufweist, das nicht nur zur Versendung der erfassten Daten des Datenerfassungsmoduls eingerichtet ist, sondern dass dieses Kommunikationsmodul auch zur Sprachkommunikation eingerichtet ist, das Kommunikationsmodul also im Wesentlichen eine dafür benötigte Telefonie-Eigenschaften mit sich bringt. So kann die qualifizierte Person mit dem Nutzer unmittelbar über die Vorrichtung zur Erfassung der Daten eine bevorzugte bi-direktionale Sprechverbindung aufbauen ohne zwingend ein separates Telefon zu benötigen.

Das erfindungsgemäße System kann weiterhin vorsehen, dass in der Datenbank für jede darin angemeldete Vorrichtung wenigstens ein Arztidentifikator bzw. eine Arztadresse gespeichert ist oder zumindest speicherbar ist, an welchen entweder die Daten aus der Datenbank durch Telekommunikation weitergeleitet werden oder an welchen eine Information darüber sendbar ist, dass Daten zum Abruf aus der Datenbank zur Verfügung stehen.

Unter einem solchen Arztidentifikator bzw. der Arztadresse kann eine Telekommunikationsadresse verstanden werden, die ein Kommunikationsgerät der qualifizierten Person in einem Netzwerk identifiziert, mittels welchem diese Person mit der Datenbank bzw. der diese verwaltenden Datenverarbeitungsanlage in Kommunikationsverbindung treten kann.

Beispielsweise können die Daten aus der Datenbank so direkt an das Kommunikationsgerät der qualifizierten Person übermittelt werden, z.B. als Email oder Anlage zu einer Email oder auf sonstige Weise oder aber eine qualifizierte Person erhält lediglich eine Nachricht darüber, dass Daten zum Abruf auf der Datenbank vorliegen, so dass die Person diesen Abruf selbst aktiv vornimmt, z.B. durch Anwahl der Datenbank über ein Kommunikationsgerät und online-Ansicht der Daten oder aber durch ein Herunterladen der Daten.

Die qualifizierte Person kann nach der entsprechenden Begutachtung der Daten durch Kommunikationsaufbau mit der Datenbank sein Begutachtungsergebnis hinterlegen oder bevorzugt vermittelt über die Datenbank eine Kommunikationsverbindung zum Nutzer aufnehmen, je nach Ergebnis der Begutachtung.

Beispielsweise kann eine qualifizierte Person (Arzt) hinsichtlich des begutachteten Datensatzes die Information in der Datenbank hinterlegen, dass die Begutachtung positiv ist, also keinerlei Krankheitsbefunde oder sonstige Handlungsanweisungen vorliegen, so dass diese Information wie vorgenannt dann von der Datenverarbeitungsanlage, welche die Datenbank verwaltet, an den Nutzer bzw. dessen genutzte Vorrichtung des Systems kommuniziert werden kann.

Anderenfalls kann die Datenbank die Kommunikationsverbindung zwischen dem Arzt und dem Nutzer entweder über die Datenbank bzw. deren Datenverarbeitungsanlage erstellen oder zumindest die Kommunikationsverbindung vermitteln.

Die Erfindung kann vorsehen, dass ein Nutzer selbst den wenigstens einen Arztidentifikator bzw. die Arztadresse ändern kann. So kann der Nutzer hierdurch beispielsweise selbst bestimmen, an welchen Arzt, z.B. den Arzt seines Vertrauens, wie den Hausarzt, die Daten zur Verfügung gestellt werden sollen.

Die Erfindung kann jedoch auch vorsehen, dass das System im Rahmen einer anonymisierten Dienstleistung verwendet wird, dass also der Nutzer weder den Arzt auswählen kann, noch der Arzt den Nutzer kennt, jedoch auch bei dieser anonymisierten Verfahrensweise ein Nutzer jeder Zeit darauf vertrauen darf, eine qualifizierte medizinisch fundierte Handlungsanweisung zu erhalten, sofern hierfür ein Bedarf besteht, ansonsten hingegen durch die positive Rückmeldung aufgrund der qualifizierten medizinischen Betrachtung beruhigt ist.

Das erfindungsgemäße System kann auch vorsehen, dass unterschiedliche Arztidentifikatoren bzw. Adressen verschiedenen auswechselbaren Datenerfassungsmodulen zugeordnet werden können. So besteht beispielsweise die Möglichkeit, je nach Art der Datenerfassung oder je nach Art der erfassten Daten, somit also im Wesentlichen abhängig von dem eingesetzten Datenerfassungsmodul einen jeweils passend qualifizierten Arzt zur Begutachtung der Daten heranzuziehen.

Hier kann es beispielsweise vorgesehen sein, dass die Vorrichtung zur Erfassung der Daten mittels des darin enthaltenen Kommunikationsmodul zusätzlich zu den Daten auch eine Kennung des verwendeten Datenerfassungsmoduls an die Datenbank übersendet, so dass in Abhängigkeit dieser an die Datenbank übermittelten Kennung ein jeweils passender Arztidentifikator bzw. Arztadresse gewählt werden kann.

In einer solchen Ausbildung des Systems kann in Verbindung mit einer Vorrichtung zur Erfassung der Daten eine einzige Datenbank genutzt werden, wobei sodann die Verteilung der Daten von unterschiedlichen Modulen in der Datenbank bzw. diese verwaltenden Datenverarbeitungsanlage organisiert ist, nämlich beispielsweise dadurch, dass die Daten je nach benutztem Datenerfassungsmodul an unterschiedliche Ärzte zur Verfügung gestellt werden.

In einer anderen Ausführung kann das System auch vorsehen, dass in der Vorrichtung für jedes von mehreren auswechselbaren Datenerfassungsmodulen eine insbesondere unterschiedliche Kommunikationsadresse einer Datenbank hinterlegt oder zumindest hinterlegbar ist, an welche das Kommunikationsmodul die Daten in Abhängigkeit des verwendeten Datenerfassungsmoduls versendet.

Beispielsweise kann hier die Kommunikationsadresse der Datenbank im jeweiligen Datenerfassungsmodul gespeichert sein, so dass sich automatisch die Kommunikationsadresse, die das Kommunikationsmodul der Vorrichtung verwendet, ändert, wenn ein anderes Datenerfassungsmodul an die Vorrichtung angesteckt wird.

Es können so im Rahmen des Systems verschiedene Datenbanken verwendet werden, die hinsichtlich des jeweils verwendeten Datenerfassungsmodules individualisiert bzw. identifiziert sind, wobei auf die jeweiligen Datenbanken sodann entsprechend spezialisierte Ärzte oder ansonsten qualifiziertes Personal Zugriff haben.

In dieser Ausgestaltung des erfindungsgemäßen Systems erfolgt demnach die arztqualifikationsbezogene Versendung der Daten bereits unmittelbar durch das Kommunikationsmodul in der Vorrichtung des Systems.

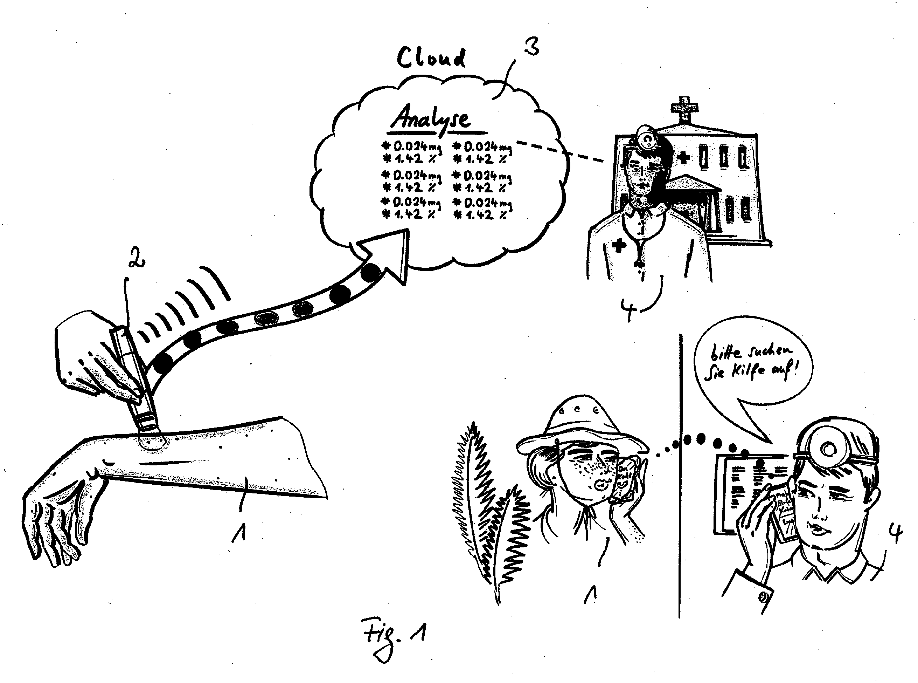

Ein Ausführungsbeispiel der Erfindung ist in der Figur 1 näher erläutert. Figur 1 zeigt, dass ein Nutzer 1 mittels der Vorrichtung 2 zur Erfassung medizinischer Daten, die hier stiftförmig ausgebildet ist, seinen Körper untersucht.

Hierfür kann an das hier untere Ende der stiftförmigen Vorrichtung ein Datenerfassungsmodul angesteckt sein, das hier beispielsweise einer Kamera entsprechen kann, so dass der Nutzer in die Lage versetzt ist, ein Hautareal hier am Unterarm optisch zu erfassen, wonach dann durch die stiftförmige Vorrichtung mit einem darin integrierten Kommunikationsmodul die Daten an eine Datenbank 3 übertragen werden, die hier als Cloud-basiert dargestellt ist, also somit beispielsweise im Internet zur Verfügung steht durch eine entsprechende Internetkommunikationsadresse.

Auf diese Daten erhält ein Arzt 4 Zugriff, beispielsweise dadurch, dass die Daten von der Datenbank an ihn weitergeleitet werden, d.h. an dessen Kommunikationsgerät oder dadurch, dass der Arzt mittels eines ihm zur Verfügung stehenden Kommunikationsgerätes die Daten aus der Datenbank aktiv abruft, z.B. nachdem er eine Information über das Vorliegen von Daten erhalten hat.

Der Arzt 4 ist nun in die Lage versetzt, aufgrund der Daten eine Diagnose zu erstellen, wobei in diesem Beispiel der Arzt 4 festgestellt hat, dass der Nutzer 1 eine medizinische Anweisung benötigt, so dass der Arzt hier über eine Sprachverbindung mit dem Nutzer 1 Kontakt aufnimmt und seine Empfehlung mitteilt. In dem hier dargestellten Beispiel erfolgt die Kontaktaufnahme zwischen zwei Telefonen, z.B. Mobiltelefonen, die beiden Personen zur Verfügung stehen.

Eine erfindungsgemäße Ausgestaltung kann jedoch auch vorsehen, dass eine Telefonverbindung direkt über die Datenerfassungsvorrichtung 2 zu einem Telefon des Arztes und zu dem Kommunikationsgerät des Arztes möglich ist, mit welchem dieser mit der Datenbank 3 in Telekommunikationsverbindung steht.

Ein Nutzer 1 kann durch die Nutzung der erfindungsgemäßen Vorrichtung 2 bzw. insgesamt des erfindungsgemäßen Systems jederzeit sicher sein, dass die von ihm in die Datenbank zur Verfügung gestellten Daten durch einen qualifizierten Arzt begutachtet werden, er somit durch die Nutzung der Vorrichtung 2 nicht lediglich eine technische Auswertung der Daten und darauf basierender ausschließlich technischer Diagnose erhält, die sich in vielen Fällen als falsch erweisen kann.

Der besondere Vorteil bei der Nutzung des erfindungsgemäßen Systems liegt darin, dass Ferndiagnosen über große Distanzen hinweg zwischen Nutzer und Arzt möglich sind.

Hierfür kann es die Erfindung allgemein vorsehen, dass das Kommunikationsmodul in der Vorrichtung ausgebildet ist, um eine Funkkommunikation über ein Mobilfunknetz, z.B. nach dem GSM Standard, zu der Datenbank aufzubauen. Von jedem Ort der Welt kann somit eine Datenübertragung von der Vorrichtung in diese Datenbank erfolgen, also unabhängig davon, wo sich der Nutzer der Vorrichtung aktuell aufhält. Eine jederzeitige qualifizierte ärztliche Diagnose kann somit einen Nutzer auf seinen Reisen begleiten.

## Patentansprüche

1. System zur Erfassung medizinischer Daten von einem Nutzer umfassend eine, bevorzugt stiftförmige Vorrichtung mit wenigstens zwei in einer Erstreckungsrichtung, bevorzugt Längserstreckungsrichtung hintereinander liegenden Komponenten, wobei eine Komponente zumindest ein Kommunikationsmodul umfasst und eine Komponente, insbesondere eine endseitig angeordnete Komponente ein bevorzugt auswechselbares Datenerfassungsmodul umfasst, mittels dem Daten, insbesondere gesundheitsspezifische Daten vom Körper des Nutzers erfassbar sind und umfassend wenigstens eine Datenbank auf einer in einem Kommunikationsnetzwerk angeordneten Datenverarbeitungsanlage, insbesondere an der das Kommunikationsmodul anmeldbar oder fix angemeldet ist, wobei die Vorrichtung eingerichtet ist, von dem Datenerfassungsmodul erfasste Daten mittels des Kommunikationsmoduls an die wenigstens eine Datenbank zu übertragen.

2. System nach Anspruch 1, **dadurch gekennzeichnet, dass** eine dem Datenerfassungsmodul endseitig gegenüberliegende Komponente eine Energieversorgung, bevorzugt auswechselbare Energieversorgung der Vorrichtung bildet.

3. System nach einem der vorherigen Ansprüche, **dadurch gekennzeichnet, dass** es eingerichtet ist, eine Begutachtung der erfassten und an die wenigstens eine Datenbank übertragenen Daten durch einen Arzt abzuwarten und ausschließlich nur bei einem in Sinne des Nutzers positiven Gutachten-Ergebnis an die Vorrichtung eine das positive Ergebnis signalisierende Nachricht zu senden, insbesondere bei einem negativen Ergebnis eine bevorzugt sprachbasierte Kommunikationsverbindung zwischen dem Nutzer und einem Arzt herzustellen.

4. System nach Anspruch 3, **dadurch gekennzeichnet, dass** die Sprachkommunikation zu der Vorrichtung durchführbar ist, in welcher das Kommunikationsmodul zusätzlich als Sprachkommunikationsmodul ausgebildet ist.

5. System nach einem der vorherigen Ansprüche, **dadurch gekennzeichnet, dass** in der Datenbank für jede Vorrichtung wenigstens ein Ärztidentifikator / Arztadresse gespeichert ist oder zumindest speicherbar, an welchen entweder die Daten durch Telekommunikation weitergeleitet werden oder an welchen ein Information darüber sendbar ist, dass Daten zum Abruf aus der Datenbank zur Verfügung stehen, insbesondere wobei die gespeicherten Identifikatoren / Adressen vom Nutzer änderbar sind.

6. System nach Anspruch 5, **dadurch gekennzeichnet, dass** unterschiedliche Artzidentifikatoren / -adressen verschiedenen auswechselbaren Datenerfassungsmodulen zugeordnet werden können, insbesondere wobei eine Vorrichtung mittels des Kommunikationsmoduls zusätzlich zu den Daten eine Kennung des verwendeten Datenerfassungsmoduls an die Datenbank übersendet, in deren Abhängigkeit ein Artzidentifikator / -adresse gewählt wird.

7. System nach einem der vorherigen Ansprüche 1 bis 4, **dadurch gekennzeichnet, dass** in der Vorrichtung für jedes von mehreren auswechselbaren Datenerfassungsmodulen eine insbesondere unterschiedliche Kommunikationsadresse einer Datenbank hinterlegt oder hinterlegbar ist, an welche das Kommunikationsmodul die Daten in Abhängigkeit des verwendete Datenerfassungsmoduls versendet.

8. System nach einem der vorherigen Ansprüche, **dadurch gekennzeichnet, dass** Datenerfassungsmodule ausgebildet sind zur Erfassung von Bildern mittels einer Kamera und/oder zur Erfassung von Urinwerten und/oder zur Erfassung von Blutwerten und/oder zur Erfassung von Blutdruckwerten und/oder zur Erfassung von Ultraschallwerten.

9. Vorrichtung, insbesondere stiftförmige Vorrichtung zur Erfassung medizinischer Daten von einem Nutzer umfassend wenigstens zwei in einer Erstreckungsrichtung, bevorzugt Längserstreckungsrichtung hintereinander liegende Komponenten, wobei eine Komponente zumindest ein Kommunikationsmodul umfasst und eine Komponente, insbesondere eine endseitig angeordnete Komponente ein bevorzugt wechselbares Datenerfassungsmodul umfasst, mittels dem Daten, insbesondere gesundheitsspezifische Daten vom Körper des Nutzers erfassbar sind und die Vorrichtung eingerichtet ist, von dem Datenerfassungsmodul erfasste Daten mittels des Kommunikationsmoduls an wenigstens eine Datenbank zur Speicherung und/oder Auswertung der Daten zu übertragen, insbesondere durch Mobilfunkkommunikation.
